(19) Europäisches Patentamt
European Patent Office
Office européen des brevets

(11) **EP 2 983 114 A1**

(12) **EUROPEAN PATENT APPLICATION**

(43) Date of publication:
**10.02.2016 Bulletin 2016/06**

(51) Int Cl.:
***G06Q 10/06*** (2012.01)

(21) Application number: **14197567.2**

(22) Date of filing: **12.12.2014**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **05.08.2014 TW 103126785**

(71) Applicant: **Environmental Protection Administration, R.O.C.(Taiwan) Taipei City 10042 (TW)**

(72) Inventors:
• **Tsai, Hung-Teh 10042 Taipei City (TW)**

• **Yang, Kai-Hsing 10042 Taipei City (TW)**
• **Chen, Shen-De 10042 Taipei City (TW)**
• **Kuan, Yung-Kai 10042 Taipei City (TW)**
• **Sun, Tung-Ching 10042 Taipei City (TW)**

(74) Representative: **Gee, Steven William et al D.W. & S.W. GEE 1 South Lynn Gardens London Road Shipston on Stour Warwickshire CV36 4ER (GB)**

(54) **Method for sorting environmental risk of abandoned plants**

(57) An environmental risk sorting method includes preparing a plant environment database for storing environmental risk data for abandoned plants; using a first risk evaluation module, based on the environmental risk data, to generate a first risk evaluation result for each abandoned plant; using the first risk evaluation result to generate an environmental site evaluation list, which in turn, generates an environmental site evaluation data of each abandoned plant and updated environmental risk data; using a second risk evaluation module, based on the environmental site evaluation data of each abandoned plant and the updated environmental risk data, to generate a second risk evaluation result for each abandoned plant; and using the second risk evaluation result for each abandoned plant to generate an investigation list as a basis for follow-up investigation.

a preliminary step: prepare a plant environment database for storing environment risk data for each abandoned plant — S10

a first risk evaluation step: generate a first risk evaluation result for each abandoned plant — S20

an environmental site evaluation step: generate an environment site evaluation data of each abandoned plant and updated the environmental risk data — S30

a second risk evaluation step: generate a second risk evaluation result for each abandoned plant — S40

a risk managing step: generate an investigation list as a basis for follow-up investigation — S50

**FIG. 1**

EP 2 983 114 A1

**Description**

CROSS-REFERENCE TO RELATED APPLICATION

This application claims the priority of Taiwanese patent application No. 103126785, filed on August 5, 2014, which is incorporated herewith by reference.

BACKGROUND OF THE INVENTION

1. Field of the Invention

The present invention relates generally to an environmental risk sorting method, and more particularly an environmental risk sorting method for monitoring abandoned plants which cause high environmental pollution.

2. The Prior Arts

Industrial pollution is one major cause for underground water pollution, since wastes or pollutants are directly or indirectly discharged into the ground without adequate treatment to remove harmful compounds. Some abandoned plants cause high environmental risk to the ground upon which the plants are built prior to shutdown or abandonment. The currently running plants need more attention since they constantly generate environmental risk if no proper environmental safety measurement is taken during the running period, and the land left by those abandoned plants may be contaminated. So that an environmental risk sorting method is required to effectively sorting each of the abandoned plants as the basis for follow-up investigation for monitoring the abandoned plants.

A conventional method of sorting abandoned plants causing high environmental risk includes fetching the recorded basic information stored within an abandoned plant and evaluating the basic information so as to generate a risk evaluation result for each abandoned plant, which in turn, produces an environmental site evaluation list. Since the environmental site evaluation list includes names of abandoned plants and location sites, it is taken as the basis for follow-up investigation and management of the location sites. It is noted the earlier basic information of the abandoned plants are recorded not in properly arranged system or lacking such as environmental risk level and hence will not be a perfect one owing to exclusion of the recent environmental site evaluation information. The environmental risk level of each abandoned plant is defined by the recorded information of the respective abandoned plant, thereby causing difficulty in obtaining the on-spot environmental risk evaluation of the abandoned plant.

Therefore, the conventional method of environmental risk sorting cannot handle a tremendously large amount of record information and incomplete information leads to waste of human labor, time, and cost, hence the management of material, needs to be upgraded urgently.

SUMMARY OF THE INVENTION

The object of the present invention is to provide an environmental risk sorting method that can eliminate the above-mentioned drawbacks resulted from the use of the conventional environmental risk sorting method and that can update a plant environment database so as generate on-spot data of each abandoned plant, thereby obtaining an environmental risk evaluation result substantially similar to the present condition so as to facilitate the follow-up management and investigation.

An environmental risk sorting method of the present invention for monitoring abandoned plants which cause high environmental pollution, includes: a preliminary step: preparing a plant environment database for storing environmental risk data for each abandoned plant, wherein the environmental risk data includes pollution potential factor data and environment factor data; a first risk evaluation step: using a first risk evaluation module, based on the environmental risk data, to generate a first risk evaluation result for each abandoned plant, wherein the first risk evaluation result includes a first risk evaluation value ($T_1$); and a first risk level defined based on the first risk evaluation value ($T_1$); an environmental site evaluation step: using the first risk evaluation result to generate an environmental site evaluation list, which in turn, generates an environmental site evaluation data of each abandoned plant and updated environmental risk data; a second risk evaluation step: using a second risk evaluation module, based on the environmental site evaluation data of each abandoned plant and the updated environmental risk data, to generate a second risk evaluation result for each abandoned plant, wherein the second risk evaluation result includes a second risk evaluation value ($T_2$) and a second risk level defined based on the second risk evaluation value ($T_2$); and a risk managing step: using the second risk evaluation result for each abandoned plant to generate an investigation list as a basis for follow-up investigation.

Preferably, the preliminary step further includes a conditional sorting procedure that consists of plant site area of the environmental risk data, plant location and storeys of each abandoned plant, and classification of abandoned plants into different groups based on usage change of each abandoned plant.

Preferably, in one embodiment of the present invention, the first risk evaluation value ($T_1$) is equivalent to a total sum of a first underground water environmental risk factor ($S_{gw, 1}$) and a first soil environmental risk factor ($S_{soil, 1}$) and is multiplied by a weighting factor F. The first underground water environmental risk factor ($S_{gw, 1}$) is computed from root mean square (RMS) of a first underground water pollution factor ($P_{gw, 1}$), an underground environmental vulnerability factor ($C_{gw}$) and an underground water pollution receptor factor ($D_{gw}$), wherein, the first underground water pollution factor ($P_{gw, 1}$) is computed from accumulated value of the environmental risk data. The first soil environmental risk factor ($S_{soil, 1}$) is computed from root mean square (RMS) of a first soil pollution potential factor ($P_{soil, 1}$), a soil environmental vulnerability factor ($C_{soil}$) and a soil pollution receptor factor ($D_{soil}$), wherein, the first soil pollution potential factor ($P_{soil, 1}$) is computed from accumulated value of the environmental risk data.

Preferably, the environmental risk data includes a pollution potential factor (P), which consists of the following factors: registered plant site area ($A_1$), plant year of running ($A_2$), former record for atmosphere, water, waste and poisonous substances ($A_3$), number of owner transfer ($A_4$), ground water pollution rate ($A_{5gw}$), soil pollution rate ($A_{5soil}$), amount of discharged polluted water into the ground ($B_{gw}$), amount of soil pollution ($B_{soil}$) and human toxicity pollution potential (HTP), which includes human toxicity groundwater pollution ($HTP_{gw}$) and human toxicity potential soil pollution ($HTP_{soil}$).

Preferably, the amount of discharged polluted water into the ground ($B_{gw}$) is the total units of polluted water discharged within a plurality of designated periods. The amount of soil pollution ($B_{soil}$) is the total units of polluted soil caused within a plurality of designated periods.

Preferably, the environmental site evaluation list includes an environmental site evaluation list of high risk level including advising to conduct on-spot environmental site evaluation for each abandoned plant; an environmental site evaluation list of middle high risk level including determining environmental site evaluation procedure of abandoned plants based on the pollution potential factor; an environmental site evaluation list of middle risk level including further soil observation and managing of abandoned plants; and an environmental site evaluation list of low risk level including no obvious public environmental risk, no need of follow-up investigation.

Preferably, the second risk evaluation value ($T_2$) is equivalent to a total sum of a second groundwater environmental risk factor ($S_{gw, 2}$) and a second soil environmental risk factor ($S_{soil, 2}$) and is multiplied by a weighting factor (F). The second groundwater environmental risk factor ($S_{gw, 2}$) is computed from root mean square (RMS) of a second underground water pollution potential factor ($P_{gw, 2}$), an underground environmental vulnerability factor ($C_{gw}$) and an underground water pollution receptor factor ($D_{gw}$), wherein the second underground water pollution potential factor ($P_{gw, 2}$) is computed from the environmental risk data and the environmental site evaluation data.

Preferably, the environmental site evaluation data includes the following factors: plant running quality factor ($I_1$), plant facilities factor ($I_2$), history of plant relocation factor ($I_3$), previous environmental spill or accident factor ($I_4$), pollution potential factor ($I_5$) and change in land or land quality inspection rating factor ($I_6$).

Preferably, the investigation list includes an investigation list of high environmental risk level including advising to conduct on-spot investigation of abandoned plants; and an investigation list of middle high environmental risk level including advising to conduct further investigation of abandoned plants.

The second risk evaluation result includes a second risk evaluation value, from which, a second risk level is defined, wherein, the investigation list is defined based on the second risk level so as to facilitate the follow-up management and investigation, which in turn, economizes human labor and time.

BRIEF DESCRIPTION OF THE DRAWINGS

The present invention will be apparent to those skilled in the art by reading the following detailed description of a preferred embodiment thereof, with reference to the attached drawings, in which:

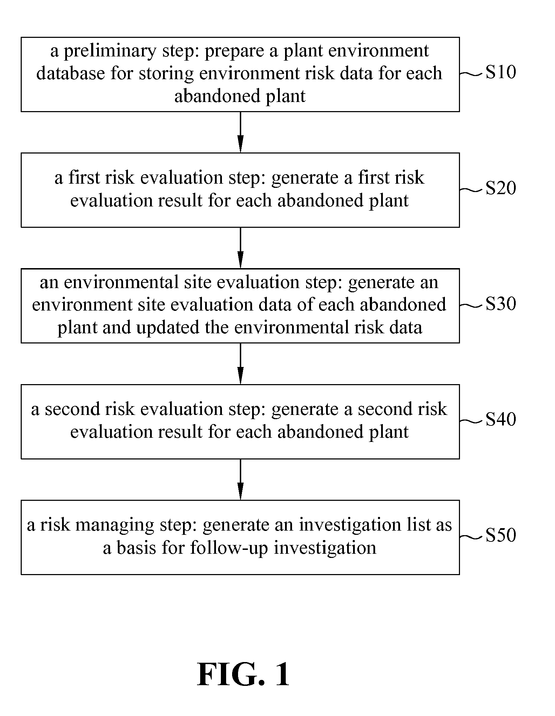

Fig. 1 illustrates a block diagram including the steps of a method of the present invention for sorting environmental risk in order to control abandoned plants which cause environmental pollution.

DETAILED DESCRIPTION OF THE PREFERRED EMBODIMENT

The accompanying drawings are included to provide a further understanding of the invention, and are incorporated in and constitute a part of this specification. The drawings illustrate embodiments of the invention and, together with the description, serve to explain the principles of the invention.

Fig. 1 illustrates a block diagram including the steps of a method of the present invention for sorting environmental risk in order to control abandoned plants which cause environmental pollution. As illustrated, in a preliminary step S10: preparing a plant environment database for storing environmental risk data for each abandoned plant, wherein the environmental risk data preferably include pollution potential factor data and environmental factor data. In one embodiment of the present invention, the pollution potential factor data consists of the following factors registered plant site

area ($A_1$); plant year of running ($A_2$); former record for atmosphere, water, waste and poisonous substances ($A_3$); number of owner transfer ($A_4$); ground water pollution rate ($A_{5gw}$); soil pollution rate ($A_{5soil}$); amount of discharged polluted water into the ground ($B_{gw}$); amount of soil pollution ($B_{soil}$); and human toxicity pollution potential (HTP), which includes human toxicity groundwater pollution ($HTP_{gw}$) and human toxicity potential soil pollution ($HTP_{soil}$). The environmental factor data preferably include infiltration amount factor ($C_1$); topography factor ($C_2$); soil medium factor ($C_3$); ventilation layer medium factor ($C_4$); hydraulic conductivity coefficient factor ($C_5$); groundwater depth factor ($C_6$); aquifer medium factor ($C_7$); population density factor ($D_1$), soil /sediment contact risk factor ($D_2$) and groundwater contact risk factor ($D_3$).

In a first risk evaluation step S20: using a first risk evaluation module to generate a first risk evaluation result for each abandoned plant, wherein the first risk evaluation result includes a first risk evaluation value ($T_1$); and a first risk level defined based on the first risk evaluation value ($T_1$). In one embodiment, the first risk evaluation module utilizes the following equation (1) to generate the first risk evaluation value ($T_1$). In this embodiment, the first risk evaluation value ($T_1$) is computed from the following equation 1:

$$T_1 = \left(S_{gw,1} + S_{soil,1}\right) \times F \qquad (1)$$

In the above equation, $S_{gw,\,1}$ is a first underground water environmental risk factor; $S_{soil,\,1}$ is a first soil environmental risk factor; and F is a weighting factor. Assuming the weighting factor F = 1.5, the first underground water environmental risk factor $S_{gw,\,1}$ and the first soil environmental risk factor $S_{soil,\,1}$ can be computed respectively from the equations (2) and (3);

$$S_{gw,1} = \sqrt{\left(P_{gw,1}^2 + C_{gw}^2 + D_{gw}^2\right)/3} \qquad (2)$$

$$S_{soil,1} = \sqrt{\left(P_{soil,1}^2 + C_{soil}^2 + D_{soil}^2\right)/3} \qquad (3)$$

In the above equation, $P_{gw,\,1}$ and $P_{soil,\,1}$ respectively represent the first underground water and potential soil pollution factors; $C_{gw}$ and $C_{soil}$ respectively stand for underground water and soil environmental vulnerability factor; and $D_{gw}$ and $D_{soil}$ respectively represent underground water and soil pollution receptor factor. According to the environmental risk data, the first underground water ($P_{gw,\,1}$) and the first potential soil pollution factor ($P_{soil,\,1}$) from the equations (2) and (3) can be computed from the following equations (4) and (5). However, owing to different running operation of the plants and time differences, the total sum for the pollution potential value of each abandoned plant is:

$$P_{gw,\,1} = \Sigma\,[(A_1 \times A_2 \times B_{gw} \times I_1 \times (1+A_3) \times I_2)\,(1+A_4 \times I_3+I_4)\times I_5 \times (1+A_{5gw}) \times HTP_{gw}] \times I_6 \qquad (4)$$

$$P_{soil,\,1} = \Sigma\,[(A_1 \times A_2 \times B_{soil} \times I_1 \times (1+A_3) \times I_2)\,(1+A_4 \times I_3+I_4)\times I_5 \times (1+A_{5soil}) \times HTP_{soil}] \times I_6 \qquad (5)$$

The underground water and soil environmental vulnerability factors $C_{gw}$ and $C_{soil}$ of the equations (2) and (3) can be computed from the following equations (6) and (7);

$$C_{gw} = C_1 + C_5 + C_6 + C_7 \qquad\qquad (6)$$

$$C_{soil} = C_2 + C_3 + C_4 \qquad\qquad (7)$$

The underground water factor ($D_{gw}$) and the soil pollution receptor factor ($D_{soil}$) of equations (2) and (3) can be computed from the following equations (8) and (9);

$$D_{gw} = D_1 + D_3 \qquad (8)$$

$$D_{soil} = D_1 + D_2 \qquad (9)$$

The corresponding value for the previous air pollution, water pollution, waste, poisonous chemical record factors (A3) are shown in table 1, the corresponding value of plant transfer time factor (A4), if transfer of the plant ownership take place once 1, if there is no transfer of plant ownership 0; the corresponding value for infiltration amount factor (C1), topographical factor (C2), soil medium factor (C3) and ventilation layer medium factor (C4) are shown in table 2; the corresponding value for hydraulic conductivity factor (C5), groundwater depth factor (C6) and aquifer medium factor (C7) are shown in table 3; and the corresponding value for population density factor (D1), soil/sediment contact risk factors (D2), and groundwater risk contact factors (D3) are shown respectively in tables 4-6. Amount of discharged polluted water into the ground (Bgw), amount of soil pollution (Bsoil), human toxicity pollution potential (HTP), previous environmental spill or accident factor (14) (A5) are shown respectively in tables 7 and 8. If there is one time occurrence of environmental spill or accident, the accident factor (14) is increased 1 while if there is no occurrence of environmental spill or accident, the accident factor (14) is equivalent to 0.

Table 1 the corresponding potential factor values for the previous air pollution, water pollution, waste, poisonous chemical record factors

| Previous air pollution, water pollution, waste, poisonous chemical record factors | $A_3$ corresponding to pollution potential factor |
|---|---|
| No record | 0 |
| The submitted previous air pollution, water pollution, waste, poisonous chemical record factors | 0.5 |
| The previous environment water pollution record | 1 |

Table 2 $C_1 \sim C_4$ transfer factor of the corresponding transfer factor value

| (C$_1$) infiltration amount (cm/year) | value | (C$_2$) topographical inclination (%) | value | (C$_3$) soil medium and pH | Value | (C$_4$) ventilation layer medium factor | Value |
|---|---|---|---|---|---|---|---|
| 0-2.5 | 0.8 | 18~99 | 0.6 | >10 | 1.2 | basalt | 1.2 |
| 2.5-5 | 1.6 | 15~18 | 1.2 | 9 | 2.4 | dolomite | 2.4 |
| 5-6.7 | 2.4 | 12~15 | 1.8 | 8 | 3.6 | marble | 3.6 |
| 6.7-8.3 | 3.2 | 10~12 | 2.4 | 7 | 4.8 | limestone | 4.8 |
| 8.3~10 | 4 | 6~10 | 3 | 6 | 6 | coal | 6 |
| 10-14 | 4.8 | 5~6 | 3.6 | 5 | 7.2 | quartz schist | 7.2 |
| 14-17 | 5.6 | 4~5 | 4.2 | 4 | 8.4 | marl | 8.4 |
| 17-25 | 6.4 | 3~4 | 4.8 | 3 | 9.6 | claystone | 9.6 |
| >25 | 8 | 2~3 | 5.4 | 2 | 10.8 | silty clay | 10.8 |
| - | - | 0~2 | 6 | 1 | 12 | clay | 12 |

Table 3 $C_5 \sim C_7$ transfer factor of the corresponding transfer factor value

| (C$_5$) hydraulic conductivity factor (m/day) | Value | (C$_6$) groundwater depth factor (m) | Value | (C$_7$) aquifer medium factor | Value |
|---|---|---|---|---|---|
| <4 | 0.6 | 0~1.5 | 10 | Quartzite, granite | 0.6 |

(continued)

| (C$_5$) hydraulic conductivity factor (m/day) | Value | (C$_6$) groundwater depth factor (m) | Value | (C$_7$) aquifer medium factor | Value |
|---|---|---|---|---|---|
| 4~8 | 1.2 | 1.5~3.5 | 9 | quartz schist, red clay | 1.2 |
| 8-12 | 1.8 | 3.5~4.5 | 8 | schist, shale | 1.8 |
| 12-20 | 2.4 | 4.5~6.75 | 7 | limestone, ansun rock | 2.4 |
| 20-28 | 3 | 6.75~9 | 6 | silty sand, silty clay | 3 |
| 28-34 | 3.6 | 9~12 | 5 | marble, coal | 3.6 |
| 34-40 | 4.2 | 12~15 | 4 | volcanic rock, sandy gravel | 4.2 |
| 40~60 | 4.8 | 15~22.5 | 3 | domolite | 4.8 |
| 60~80 | 5.4 | 22.5~30 | 2 | basalt | 5.4 |
| >80 | 6 | >30 | 1 | boulder | 6 |

Table 4 population density factor and the corresponding factor value

| (D$_1$) density (person/km2) | value |
|---|---|
| ≦ 500 | 1 |
| 500~1000 | 2 |
| 1000~5000 | 4 |
| 5000~12000 | 6 |
| 12000~47000 | 8 |
| ≧ 47000 | 10 |

Table 5 soil/sediment contact risk factors and the corresponding factor value

| Soil implementation level | Receptor | (D$_2$) soil/sediment contact risk factors |
|---|---|---|
| Agriculture | Farmer: resident | 20 |
| Aquaculture | aquaculture industry: resident | 16 |
| Forest | Visitor | 2 |
| Traffic | Visitor | 0 |
| Reservoir | Farmer: resident | 16 |
| Groundwater well | Farmer: resident | 0 |
| Water conservancy | staff: visitor | 4 |
| Residential house | resident, children | 6 |
| Business | staff: resident | 4 |
| Culture & education | Children : resident | 6 |
| Building | Staff: resident | 4 |
| Environment | Staff: resident | 4 |
| Industry | Staff: resident | 4 |
| Medical treatment | Staff: resident | 4 |
| Public | Staff: resident | 4 |

(continued)

| Soil implementation level | Receptor | $(D_2)$ soil/sediment contact risk factors |
|---|---|---|
| Recreation | Visitor | 2 |

Table 6 groundwater risk contact potential factors and the corresponding factor value

| Soil implementation classification | Receptor | $(D_3)$ groundwater risk contact potential factor |
|---|---|---|
| Agriculture | Farmer : resident | 6 |
| Aquaculture | Aquaculture Industry: resident | 6 |
| Forest | Visitor | 2 |
| Traffic | Visitor | 0 |
| Reservoir | Farmer : resident | 6 |
| Groundwater well | Farmer : resident | 20 |
| Water conservancy | Staff : visitor | 6 |
| Residential housing | Resident : children | 20 |
| Business | Staff : resident | 6 |
| Culture and Education | Children: Resident | 6 |
| Building | Staff : resident | 6 |
| Environment | Staff : resident | 6 |
| Industry | Staff : resident | 6 |
| Medical treatment | Staff : resident | 6 |
| Public | Staff : resident | 6 |
| Recreation | Visitor | 2 |

Table 7 amount of polluted water discharged into the ground and soil pollution caused thereby

| Category Code | Type of industry | Discharge of polluted water into the ground (in unit) | | Soil pollution caused thereby (in unit) | |
|---|---|---|---|---|---|
| | | Before yr 94 $B_{gw94}$ | After yr 94 $B_{gw94}$ | Before yr 94 $B_{soil94}$ | After yr 94 $B_{soil94}$ |
| 13 | Fur and Leather Product Manufacturing | 1.49E-04 | 5.77E-05 | 3.11E-04 | 6.68E-05 |
| 14 | Wood and bamboo manufacturing | 1.18E-06 | 1.00E-06 | 1.26E-05 | 3.98E-06 |
| 17 | Petroleum and Coal Products | 9.10E-06 | 2.95E-05 | 2.39E-05 | 4.04E-05 |
| 1810 | Basic Chemical Industries | 5.07E-05 | 1.24E-04 | 6.85E-05 | 9.89E-05 |
| 1820 | Petrochemicals | 6.58E-05 | 2.62E-04 | 8.88E-05 | 2.08E-04 |
| 1830 | Fertilizer Manufacturing | 3.06E-05 | 3.81E-05 | 2.55E-05 | 3.18E-05 |
| 1841 | Synthetic resin industry | 2.51E-05 | 5.28E-05 | 8.61E-05 | 1.81E-04 |
| 1842 | Synthetic Rubber Manufacturing | 9.65E-05 | 2.09E-04 | 1.30E-04 | 1.66E-04 |
| 1850 | Rayon manufacturing | 7.11E-04 | 5.89E-04 | 1.66E-04 | 1.37E-04 |
| 1910 | Pesticides and Herbicides manufacturing | 5.06E-07 | 8.33E-07 | 7.26E-06 | 1.20E-05 |

(continued)

| Category Code | Type of industry | Discharge of polluted water into the ground (in unit) | | Soil pollution caused thereby (in unit) | |
|---|---|---|---|---|---|
| | | Before yr 94 $B_{gw94}$ | After yr 94 $B_{gw94}$ | Before yr 94 $B_{soil94}$ | After yr 94 $B_{soil94}$ |
| 1920 | Paint, dye and pigment manufacturing | 1.08E-04 | 1.01E-04 | 1.45E-04 | 8.02E-05 |
| 22 | Plastic Products Manufacturing | 1.54E-05 | 1.55E-05 | 1.19E-04 | 3.41E-05 |
| 24 | Basic metal industries | 7.88E-04 | 1.10E-03 | 9.78E-04 | 1.32E-03 |
| 2543 | Metal Heat Treatment Industry | 2.52E-05 | 3.36E-05 | 6.66E-05 | 4.58E-05 |
| 2544 | Metal surface treatment industry | 3.59E-05 | 2.51E-05 | 9.50E-05 | 3.42E-05 |
| 26 | Electronic Components Manufacturing | 2.22E-05 | 3.46E-05 | 5.48E-05 | 4.05E-05 |
| 27 | Computer communications and audio-visual and electronic products | 3.63E-05 | 4.38E-05 | 8.94E-05 | 5.13E-05 |
| 28 | Power Supplies and Equipment Manufacturing | 2.97E-05 | 2.42E-05 | 1.12E-04 | 3.88E-05 |
| 381 | Waste collection | 2.09E-03 | 8.27E-04 | 2.04E-03 | 8.14E-04 |
| 382 | Waste Treatment Industry | 8.73E-04 | 4.09E-04 | 8.48E-04 | 4.03E-04 |

Table 8 potential toxicity weights caused by each industry

| Category Code | Type of industry | $HTP_{gw}$ | $HTP_{soil}$ |
|---|---|---|---|
| 13 | Fur and Leather Product Manufacturing | 3.3E+01 | 4.2E-02 |
| 14 | Wood and bamboo manufacturing | 1.5E+02 | 2.9E-01 |
| 17 | Petroleum and Coal Products | 3.9E+01 | 2.2E-01 |
| 1810 | Basic Chemical Industries | 2.6E+02 | 3.8E-01 |
| 1820 | Petrochemicals | 3.5E+02 | 3.1E-01 |
| 1830 | Fertilizer Manufacturing | 1.1E-01 | 7.2E-03 |
| 1841 | Synthetic resin industry | 1.0E+02 | 1.6E-01 |
| 1842 | Synthetic Rubber Manufacturing | 2.1E+02 | 3.1E-01 |
| 1850 | Rayon manufacturing | 1.8E+00 | 2.1E-02 |
| 1910 | Pesticides and Herbicides manufacturing | 1.2E+03 | 4.5E-01 |
| 1920 | Paint, dye and pigment manufacturing | 2.1E+02 | 3.1E-01 |
| 22 | Plastic Products Manufacturing | 1.5E+02 | 3.0E-01 |
| 24 | Basic metal industries | 1.5E+02 | 3.0E-01 |
| 2543 | Metal Heat Treatment Industry | 1.5E+02 | 2.9E-01 |
| 2544 | Metal surface treatment industry | 1.5E+02 | 2.9E-01 |
| 26 | Electronic Components Manufacturing | 5.3E+01 | 2.8E-01 |
| 27 | Computer communications and audio-visual and electronic products | 5.3E+01 | 2.8E-01 |
| 28 | Power Supplies and Equipment Manufacturing | 1.5E+02 | 3.1E-01 |
| 381 | Waste collection | 1.3E+03 | 6.1E-01 |

(continued)

| Category Code | Type of industry | $HTP_{gw}$ | $HTP_{soil}$ |
|---|---|---|---|
| 382 | Waste Treatment Industry | 1.3E+03 | 6.1E-01 |

The first risk evaluation value ($T_1$) of the abandoned plants, and the accumulated of first risk level are shown in table 9. The high risk level and scope is 90% greater than the accumulated first risk evaluation value, i.e., the first risk evaluation value ($T_1$) ranges 60~100; the middle high risk level is 50%~90 greater than the accumulated first risk evaluation value, i.e., the first risk evaluation value ($T_1$) ranges 40-59, the middle risk level is greater than 10~50% of the accumulated first risk evaluation value, i.e., the first risk evaluation value ($T_1$) ranges 30-39 while the low risk level is 10% smaller than the accumulated first risk evaluation value, i.e., the first risk evaluation value ($T_1$) ranges 0-29.

Table 9 The first risk evaluation value, accumulated risk and risk level of abandoned plants

| First risk level | Accumulated risk (%) | First risk evaluation value |
|---|---|---|
| Low risk level | 0 | 20.86 |
| Middle risk level | 10 | 32.32 |
| | 20 | 34.21 |
| | 30 | 36.19 |
| | 40 | 37.94 |
| Middle high risk level | 50 | 41.53 |
| | 60 | 50.23 |
| | 70 | 53.37 |
| | 80 | 56.41 |
| High risk level | 90 | 59.55 |
| | 100 | 78.13 |

In the environmental site evaluation step S30, the investigator fetches the environmental site evaluation data and the updated environmental risk data. Firstly, the environmental site evaluation list is defined based on the first risk level and the first risk evaluation value ($T_1$). That is if the first risk evaluation value ($T_1$) ranges 60-100, an environmental site evaluation list of high risk level is defined. If the first risk evaluation value ($T_1$) ranges 40-59, an environmental site evaluation list of middle high risk level is defined. If the first risk evaluation value ($T_1$) ranges 30-39, an environmental site evaluation list of middle risk level is defined. If the first risk evaluation value ($T_1$) ranges 0-29, an environmental site evaluation list of low risk level is defined. Afterward, the abandoned plants in the environmental site evaluation list of the high or middle risk level are further classified again so as to update the environmental site evaluation data and the environmental risk data, wherein the environmental site evaluation data includes the following factors: plant running quality factor ($I_1$), plant facilities factor ($I_2$), history of plant relocation factor ($I_3$), previous environmental spill or accident factor ($I_4$), pollution potential factor ($I_5$) and change in land or land quality inspection rating factor ($I_6$).

In the second risk evaluation step S40: the investigator fetches the updated environmental risk data and the environmental site evaluation data via the second risk evaluation module as in the first risk evaluation step S30 to compute out the second risk evaluation result for each abandoned plant, wherein the second risk evaluation result in fact is the second risk evaluation value ($T_2$), from which the second risk level is defined. The second risk evaluation value ($T_2$) can be computed from the following equation 10:

$$T_2 = \left(S_{gw,2} + S_{soil,2}\right) \times F \qquad (10)$$

In the above equation, $S_{gw,2}$ is a second groundwater environmental risk factor; $S_{soil,2}$ is the second soil environmental risk factor; F is the weighting factor, in one embodiment it is assumed as 1.5. The second groundwater environmental risk factor $S_{gw,2}$ and the second soil environmental risk factor $S_{soil,2}$ of the above equation (10) can be computed from the following equations (11) and (12) respectively:

$$S_{gw,2} = \sqrt{\left(P_{gw,2}^2 + C_{gw}^2 + D_{gw}^2\right)/3} \qquad (11)$$

$$S_{soil,2} = \sqrt{\left(P_{soil,2}^2 + C_{soil}^2 + D_{soil}^2\right)/3} \qquad (12)$$

In the above equation, $P_{gw,2}$ and $P_{soil,2}$ respectively represent the second ground water and the second soil pollution potential factor; $C_{gw}$ and $C_{soil}$ respectively represent ground water and soil environmental vulnerability factor; $D_{gw}$ and $D_{soil}$ respectively represent ground water and soil pollution receptor factors. The second ground water $P_{gw,2}$ and the second soil pollution potential factor $P_{soil,2}$ of the above equations (11) and (12) are based on the updated environmental risk data and the environmental site evaluation data and can be computed from the following equations (13) and (14), wherein,, owing to different running operation of the plants and time differences, the total sum for the pollution potential value of each abandoned plant is:

$$\boldsymbol{P_{gw,\ 2}} = \Sigma \left[(A_1 \times A_2 \times B_{gw} \times \boldsymbol{I_1} \times (1+A_3) \times \boldsymbol{I_2}) \ (1+A_4 \times \boldsymbol{I_3} + \boldsymbol{I_4}) \times \boldsymbol{I_5} \times (1+A_{5gw}) \times HTP_{gw}\right] \times \boldsymbol{I_6}$$
$$(13)$$

$$\boldsymbol{P_{soil,\ 2}} = \Sigma \left[(A_1 \times A_2 \times B_{soil} \times \boldsymbol{I_1} \times (1+A_3) \times \boldsymbol{I_2}) \ (1+A_4 \times \boldsymbol{I_3} + \boldsymbol{I_4}) \times \boldsymbol{I_5} \times (1+A_{5soil}) \times HTP_{soil}\right] \times \boldsymbol{I_6} \qquad (14)$$

The evaluated ways of the updated environmental risk data is the same as the previous environmental risk data. The environmental site evaluation factor data includes the following factors: plant running quality factor ($I_1$), plant facilities factor ($I_2$), history of plant relocation factor ($I_3$), previous environmental spill or accident factor ($I_4$), pollution potential factor ($I_5$) and change in land or land quality inspection rating factor ($I_6$) obtained from Table 10. An important aspect to note is that for the common knowledge in this technical field, Table 10 clearly shows the sorting level and recycling of the value of environmental site evaluation is likely happened. Preferably, the value of environmental site evaluation data of the present invention is determined by professional persons, who possess the following qualifications (1) professional technicians possessing environment engineering, applied geology, geotechnical engineering practice license; (2) persons having more than three years, soil or groundwater pollution investigation after completing at least Master Degree from public or private university or independent college recognized by the Ministry of Education or foreign university in engineering or site assessment of relevant work experience; and (3) persons having more than five years in soil or groundwater pollution investigation after graduating in the engineering, agriculture, medicine field from public or private university recognized by the Ministry of Education or foreign university or relevant work experience. More preferably, the previously mentioned persons should have been trained through the government agency in charge of environmental protection, and have passed or completed the test concerning environmental protection.

Table 10 factor correction table for sorting abandoned plant through the second risk evaluation

| Name of factor | Amended code | Description | | Difference |
|---|---|---|---|---|
| Operation quality of plant | I₁ | To further control operation quality of plants via the environmental site evaluation data, the evaluation value is set in advance 1: | | New |
| | | $I_1$ | Highlight | |
| | | 1 | No specific plant data except the speculated pollution generating substances used by the plant owner or list of some control of pollutants | |
| | | 0.8 | The controlled pollutants include such as dioxins, PCBs, pesticides, chlorinated organics, which are difficult to break down according to EPA method of soil pollution in the environment | |
| | | 0.6 | controlled pollutants include such as heavy metals, volatile organic solvents, oil of non-pollution of soil according to EPA regulated law | |
| | | 0.4 | Plant operation includes pollutants, like volatile organic solvents, semi-volatile organic solvents, toxic chemicals, and other environmental hormones which are excluded from EPA regulated law | |
| | | 0.1 | No pollutants found during operation of plant | |

| Process devices rating | $I_2$ | To further control process devices of plants via the environmental site evaluation data, the evaluation value is set in advance 1: | New |
|---|---|---|---|
| | | | |

To further control process devices of plants via the environmental site evaluation data, the evaluation value is set in advance 1:

| $I_2$ | Highlight |
|---|---|
| 2.0 | Process devices include wet operation, like wastewater treatment facilities, underground storage tanks, discharge ditches or pipes, sumps or wells |
| 1.6 | Process devices includes ground storage tank, tank and other facilities for loading liquid, raw materials, semi-finished or finished products |
| 1.4 | Process devices generates dust, slag, bottom ash and other waste containing heavy metals |
| 0.5 | Process devices use organic solvents for cleaning and wiping, dilution and coating, but no wastewater generated |
| 0.1 | No significant pollution potential, such as plastic injection, wood cutting, assembling parts found |

**Process devices rating** — $I_2$ — **New**

To further understand history of plant relocation via the environmental site evaluation data, the evaluation value is set in advance 1:

| $I_3$ | Highlight |
|---|---|
| 1 | No relocation found |
| 0 | Located at its initial spot, no relocation recorded |

**history of plant relocation** — $I_3$ — **New**

To further understand safety of working environment via the environmental site evaluation data, the evaluation value is set in advance 0:

| $I_4$ | Highlight |
|---|---|
| 1 | Each leak of pollutant, outbreak of fire or accident in short time results in 1 score |
| 0 | No record of leak of pollutant, outbreak of fire or any accident |

**Occurrence of pollution or accident** — $I_4$ — **New**

| | | To further understand the status of plant pollution via the environmental site evaluation data, the evaluation value is set in advance 1: | |
|---|---|---|---|
| Potential pollution rating | $I_5$ | <table><tr><td>$I_5$</td><td>Highlight</td></tr><tr><td>2.0</td><td>On-spot inspection finds dumping, broken or damaged tank, tank or sump; or dust collection, slag, and other spilling, sediment spreading circumstances</td></tr><tr><td>1.6</td><td>On-spot inspection finds bare ground, abnormal color, projections, and piles of unknown substances or soil</td></tr><tr><td>1.4</td><td>cracks in cement floor, patching on floor, water pool or pond</td></tr><tr><td>0.5</td><td>The initial plant is brick or iron building, no demolition or alteration records, no obvious abnormalities</td></tr><tr><td>0.1</td><td>The initial plant is reinforced concrete buildings, no demolition or alteration record, no significant pollution found.</td></tr></table> | New |
| soil or land quality | $I_6$ | To further understand the soil quality of plant via the environmental site evaluation data, the evaluation value is set in advance 1:<table><tr><td>$I_6$</td><td>Highlight</td></tr><tr><td>0.5</td><td>Under environment evaluation by authority concerned, no record of soil or land pollution found in the plant.</td></tr><tr><td>0.1</td><td>Inspection of soil or land carried out under regulations 8 and 9, no record of soil or land pollution found in the plant.</td></tr><tr><td>0.01</td><td>The plant's location was a former living community and hence undergone land renovation</td></tr></table> | New |

In the risk managing step S50, an investigation list of each abandoned plant is generated based on the second risk evaluation result as a basis for follow-up investigation. Firstly, the investigation list is defined based on the second risk level and the second risk evaluation value ($T_2$), that is a high risk investigation list is defined when the second risk evaluation value ($T_2$) ranges 60~100%, a middle high risk investigation list is defined when the second risk evaluation value ($T_2$) ranges 40~59%; a middle risk investigation list is defined when the second risk evaluation value ($T_2$) ranges 30~39% and a low risk investigation list is defined when the second risk evaluation value ($T_2$) ranges 0~29%. Afterward, based those investigation lists, the following Table 11 is drawn as a basis for follow-up investigation, wherein plant A...I indicates code for abandoned plant.

Table 11 investigation list and managing plan

| List | Abandoned plant | Follow-up managing plan |
|---|---|---|
| Low risk investigation list | Plant A | No obvious public environmental risk, no need of follow-up investigation. |
| Middle risk investigation list | Plant D, F | No obvious public environmental risk, soil follow-up investigation is needed. |
| Middle high risk investigation list | Plant B, C | Based on soil and groundwater pollution potential factor value, plant evaluation is required. |
| High risk investigation list | Plant E, G, H, I | Need on-spot site investigation |

In one embodiment of the present invention, the preliminary step further includes a conditional sorting procedure that consists of plant site area of the environmental risk data, plant location and storeys of each abandoned plant, and classification of abandoned plants into different groups based on usage change of each abandoned plant so as to serve as follow-up investigation for the specific group with lesser pollution, thereby avoiding the undesired investigation process. For instance, the abandoned plant concerns metal industry, having a surface area 1378 square meter, registered in 1995, abandoned in 2003, the registered address is No. XXX, 4F, X road and etc. According to the environmental risk data, the above abandoned plant is included in the middle high risk list based on the first risk evaluation result, a further investigation is needed. Since the abandoned plant is located at $4^{th}$ floor, there should not be any soil or water pollution potential data. Owing to the merit of conditional sorting procedure, the above mentioned plants can be re-classified into another groups, thereby avoiding the environmental risk evaluation resulted from the first risk evaluation list.

In one embodiment of the present invention, under different environment protection laws of different periods, the amount of discharged polluted water into the ground ($B_{gw}$) and the amount of soil pollution ($B_{soil}$) caused thereby by a certain type industry are relatively high or low. Therefore, in order to avoid the mis-evaluation of the discharged pollutants calculated by average mean of the total years, the abandoned plants are classified into different groups based on discharged amount of polluted water into the ground ($B_{gw}$) and the amount of soil pollution ($B_{soil}$) according to the designated time and year. For instance, the person concerned wishes to investigate one particular plant abandoned before the year 2005, the amount of discharged polluted water into the ground ($B_{gw,\ before\ 2005}$, $B_{gw,\ after\ 2005}$) and the amount of soil pollution ($B_{soil,\ before\ 2005}$, $B_{soil,\ after\ 2001}$) caused by one specified industry before and after January 1, 2005 are evaluated as the basis of the follow-up investigation so that he can evaluate the amount of polluted water discharged into the ground ($B_{gw}$) and the amount of soil pollution ($B_{soil}$) caused by that specified industry or plant abandoned after the year 2005.

The environmental site evaluation step S30 includes fetching the on-spot environmental risk data of each abandoned plant and since the environmental risk data within the environment plant database is inconsistent with the on-sport environmental risk data owing to incomplete record of the abandoned plant. Under this condition, the environmental site evaluation step includes fetching the on-spot environmental risk data of each abandoned plant and updating the environmental risk data within the environment plant database, wherein the on-spot environmental risk data includes the on-spot pollution potential factor data and the on-spot environmental factor data. Preferably, the on-spot pollution potential factor data includes the on-spot plant area ($A_1$'), the on-spot plant running yea ($A_2$'), the previous air pollution, water pollution, waste, poisonous record factors ($A_3$'), the on-spot plant transfer time factor ($A_4$'), the on-spot potential ground water pollution ($A_{5gw}$') of a respective plant ($A_{5gw}$'), the on-spot potential soil pollution ($A_{5soil}$'), the on-spot amount of discharged polluted water into the ground ($B_{gw}$'), the on-spot amount of soil pollution ($B_{soil}$'), the on-spot human toxicity potential groundwater pollution ($HTP_{gw}$'), and the on-spot human toxicity potential soil pollution ($HTP_{soil}$'). The on-spot environmental risk data preferably include the on-spot infiltration amount factor ($C_1$'), the on-spot topography factor ($C_2$'), the on-spot soil medium factor ($C_3$'), the on-spot ventilation layer medium factor ($C_4$'), the on-spot hydraulic conductivity coefficient factor ($C_5$'), the on-spot groundwater depth factor ($C_6$'), the on-spot aquifer medium factor ($C_7$'), the on-spot population density factor ($D_1$') and the on-spot soil /sediment contact risk factor ($D_2$').

As explained above, the following advantages are achieved. The investigator is capable of fetching the first environmental risk evaluation list based on the conditional sorting procedure, thereby narrowing the scope of the environmental site risk evaluation and thus obtaining the environmental site evaluation list including the environment evaluation data and the updated environmental risk data. Afterward, he is capable of fetching the second environmental risk evaluation list based on the environment evaluation data and the updated environmental risk data such that the second environmental risk evaluation list is generally similar to the present condition of each abandoned plant, thereby facilitating the sorting environmental risk for monitoring abandoned plants.

Although the present invention has been described with reference to the preferred embodiments thereof, it is apparent to those skilled in the art that a variety of modifications and changes may be made without departing from the scope of the present invention which is intended to be defined by the appended claims.

**Claims**

1. A method for sorting environmental risk for monitoring abandoned plants which cause environmental pollution, comprising:

   a preliminary step: preparing a plant environment database for storing environmental risk data for each abandoned plant;
   a first risk evaluation step: using a first risk evaluation module, based on said environmental risk data, to generate a first risk evaluation result for each abandoned plant; **characterized in that** the method further comprising:
   an environmental site evaluation step: using said first risk evaluation result to generate an environmental site evaluation list, which in turn, generates an environmental site evaluation data of each abandoned plant and updated environmental risk data;
   a second risk evaluation step: using a second risk evaluation module, based on said environmental site evaluation data of each abandoned plant and said updated environmental risk data, to generate a second risk evaluation result for each abandoned plant; and
   a risk managing step: using said second risk evaluation result for each abandoned plant to generate an investigation list as a basis for follow-up investigation.

2. The environmental risk sorting method according to claim 1, wherein said preliminary step further includes a conditional sorting procedure that consists of plant site area of said environmental risk data, plant location and storeys of each abandoned plant, and classification of abandoned plants into different groups based on usage change of each abandoned plant.

3. The environmental risk sorting method according to claim 1, wherein said environmental risk data includes pollution potential factor data and environmental factor data.

4. The environmental risk sorting method according to claim 1 or 2, wherein said first risk evaluation result includes

   a first risk evaluation value ($T_1$); and
   a first risk level defined based on said first risk evaluation value ($T_1$); wherein said environmental site evaluation list is defined by said first risk level.

5. The environmental risk sorting method according to claim 4, wherein said first risk evaluation value ($T_1$) is equivalent to a total sum of a first underground water environmental risk factor ($S_{gw, 1}$) and a first soil environmental risk factor ($S_{soil, 1}$) and is multiplied by a weighting factor F.

6. The environmental risk sorting method according to claim 5, wherein said first underground water environmental risk factor ($S_{gw, 1}$) is computed from root mean square (RMS) of a first underground water pollution factor ($P_{gw, 1}$), an underground environmental vulnerability factor ($C_{gw}$) and an underground water pollution receptor factor ($D_{gw}$), wherein, the first underground water pollution factor ($P_{gw, 1}$) is computed from accumulated value of said environmental risk data.

7. The environmental risk sorting method according to claim 5, wherein said first soil environmental risk factor ($S_{soil, 1}$) is computed from root mean square (RMS) of a first soil pollution potential factor ($P_{soil, 1}$), a soil environmental vulnerability factor ($C_{soil}$) and a soil pollution receptor factor ($D_{soil}$), wherein, said first soil pollution potential factor ($P_{soil, 1}$) is computed from accumulated value of said environmental risk data.

8. The environmental risk sorting method according to claim 1, wherein said environmental risk data includes a pollution potential factor data (P), which consists of the following factors:

   registered plant site area ($A_1$);
   plant year of running ($A_2$);
   former record for atmosphere, water, waste and poisonous substances ($A_3$);
   number of owner transfer ($A_4$);

ground water pollution rate ($A_{5gw}$);
soil pollution rate ($A_{5soil}$);
amount of discharged polluted water into the ground ($B_{gw}$);
amount of soil pollution ($B_{soil}$); and
human toxicity pollution potential (HTP), which includes human toxicity groundwater pollution ($HTP_{gw}$) and human toxicity potential soil pollution ($HTP_{soil}$).

9. The environmental risk sorting method according to claim 8, wherein said amount of discharged polluted water into the ground ($B_{gw}$) is the total units of polluted water discharged within a plurality of designated periods.

10. The environmental risk sorting method according to claim 8, wherein said amount of soil pollution ($B_{soil}$) is the total units of polluted soil caused within a plurality of designated periods.

11. The environmental risk sorting method according to claim 1, wherein said environmental site evaluation list includes

an environmental site evaluation list of high risk level including advising to conduct on-spot environmental site evaluation for each abandoned plant;
an environmental site evaluation list of middle high risk level including determining environmental site evaluation procedure of abandoned plants based on said pollution potential factor;
an environmental site evaluation list of middle risk level including further soil observation and managing of abandoned plants; and
an environmental site evaluation list of low risk level including no obvious public environmental risk, no need of follow-up investigation

12. The environmental risk sorting method according to claim 1 or 2, wherein said second risk evaluation result includes

a second risk evaluation value ($T_2$); and
a second risk level defined based on said second risk evaluation value ($T_2$), wherein said investigation list is defined based on said second risk level.

13. The environmental risk sorting method according to claim 12, wherein said second risk evaluation value ($T_2$) is equivalent to a total sum of a second groundwater environmental risk factor ($S_{gw,\,2}$) and a second soil environmental risk factor ($S_{soil,\,2}$) and is multiplied by a weighting factor (F).

14. The environmental risk sorting method according to claim 13, wherein said second groundwater environmental risk factor ($S_{gw,\,2}$) is computed from root mean square (RMS) of a second underground water pollution potential factor ($P_{gw,\,2}$), an underground environmental vulnerability factor ($C_{gw}$) and an underground water pollution receptor factor ($D_{gw}$), wherein said second underground water pollution potential factor ($P_{gw,\,2}$) is computed from said environmental risk data and said environmental site evaluation data.

15. The environmental risk sorting method according to claim 13, wherein said second soil environmental risk factor ($S_{soil,\,2}$) is computed from root mean square (RMS) of a second soil pollution potential factor ($P_{soil,\,2}$), a soil environmental vulnerability factor ($C_{soil}$) and a soil pollution reception factor ($D_{soil}$), wherein said second soil pollution potential factor ($P_{soil,\,2}$) is computed from said environmental risk data and said environmental site evaluation data.

16. The environmental risk sorting method according to claim 12, wherein said environmental site evaluation data includes the following factors:

plant running quality factor ($I_1$);
plant facilities factor ($I_2$);
history of plant relocation factor ($I_3$);
previous environmental spill or accident factor ($I_4$);
pollution potential factor ($I_5$); and
change in land or land quality inspection rating factor ($I_6$).

17. The environmental risk sorting method according to claim 12, wherein said investigation list includes

an investigation list of high environmental risk level including advising to conduct on-spot investigation of abandoned plants; and
an investigation list of middle high environmental risk level including advising to conduct further investigation of abandoned plants.

18. The environmental risk sorting method according to claim 1, wherein said environmental site evaluation step further includes fetching on-spot environmental risk data of each abandoned plant based on said environmental site evaluation list, updating data within said plant environment database based on said on-spot environmental risk data so as to obtain updated environmental risk data.

a preliminary step: prepare a plant environment database for storing environment risk data for each abandoned plant ~S10

a first risk evaluation step: generate a first risk evaluation result for each abandoned plant ~S20

an environmental site evaluation step: generate an environment site evaluation data of each abandoned plant and updated the environmental risk data ~S30

a second risk evaluation step: generate a second risk evaluation result for each abandoned plant ~S40

a risk managing step: generate an investigation list as a basis for follow-up investigation ~S50

# FIG. 1

**EUROPEAN SEARCH REPORT**

Application Number

EP 14 19 7567

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| | The claimed subject-matter, with due regard to the description and drawings in accordance with Art. 92 EPC, relates to processes comprised in the list of subject-matter and activities excluded from patentability under Art. 52(2) and (3) EPC, namely a method for performing a mental act. The information technology employed as an enabler for carrying out said processes is limited to a computing module and a database. Its use for carrying out non-technical processes forms part of common general knowledge and it was widely available to everyone at the date of filing of the present application. No documentary evidence is therefore considered necessary. (See Official Journal EPO 11/2007, pages 592ff and 594ff)<br>----- | | INV.<br>G06Q10/06 |

TECHNICAL FIELDS
SEARCHED (IPC)

G06Q

The present search report has been drawn up for all claims

| Place of search | Date of completion of the search | Examiner |
|---|---|---|
| The Hague | 1 December 2015 | Sigolo, Alessandro |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons
.............................................................................
& : member of the same patent family, corresponding
document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- TW 103126785